(19) Europäisches Patentamt
European Patent Office
Office européen des brevets

(11) Numéro de publication : **0 078 785**
**B1**

(12) **FASCICULE DE BREVET EUROPÉEN**

(45) Date de publication du fascicule du brevet : **03.04.85**

(21) Numéro de dépôt : **82870062.5**

(22) Date de dépôt : **03.11.82**

(51) Int. Cl.[4] : **C 07 C 53/128**, C 07 C 51/41, C 07 C101/06, C 07 C101/24, C 07 D233/64, A 61 K 31/195

(54) **Sels d'acide valproïque, leur préparation et leur utilisation.**

(30) Priorité : **04.11.81 LU 83729**

(43) Date de publication de la demande : **11.05.83 Bulletin 83/19**

(45) Mention de la délivrance du brevet : **03.04.85 Bulletin 85/14**

(84) Etats contractants désignés : **AT BE CH DE FR GB IT LI NL SE**

(56) Documents cités :
**FR-A- 1 012 750**
**US-A- 3 024 272**
**US-A- 3 325 361**
**CHEMICAL ABSTRACTS, vol. 93, no. 5, 4 août 1980, page 113, no. 37480c, Columbus, Ohio, USA, S.F. SAAD et al.: "Modification of the anticonvulsant action of sodium valproate by the concomitant administration of aminooxyacetic acid (GABA) content in mice"**
**CHEMICAL ABSTRACTS, vol. 96, no. 19, 10 mai 1982, page 69, no. 155386m, Columbus, Ohio, USA, A. SULCOVA et al.: "Effects of calcium valproate and aminooxyacetic acid on agonistic behavior in mice"**
**Le dossier contient des informations techniques présentées postérieurement au dépôt de la demande et ne figurant pas dans le présent fascicule.**

(73) Titulaire : **"PHARLYSE"**
**Boulevard Royal, 2**
**Luxembourg (LU)**

(72) Inventeur : **Deboeck, Arthur Marie**
**61 A Steenweg op Edingen**
**B-1540 Herne (BE)**

(74) Mandataire : **Schmitz, Yvon et al**
**Bureau Gevers S.A. 7, rue de Livourne Bte 1**
**B-1050 Bruxelles (BE)**

**EP 0 078 785 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention est relative à des sels pharmaceutiquement utilisables, solubles dans l'eau, de l'acide valproïque.

L'acide valproïque ou acide 2-n propylpentanoïque est un médicament bien connu, particulièrement pour ses propriétés anticonvulsivantes. Il présente néanmoins les inconvénients d'être à la fois liquide et pratiquement insoluble dans l'eau, ce qui gêne son utilisation dans la plupart des formes pharmaceutiques. Des sels de métaux alcalins d'acide valproïque ont été préparés afin de remédier à ces inconvénients, mais ceux-ci, bien que solubles et solides, ont le désavantage d'apporter avec eux des ions alcalins, notamment du sodium, qui sont souvent contre-indiqués.

On citera, à cet effet, le brevet US-A-3.325.361 relatif à l'utilisation de sels de métaux alcalins d'acide valproïque à titre de médicaments antiépileptiques. L'inconvénient majeur de ces sels, dû au fait qu'ils doivent dans la plupart des cas être utilisés à des doses chroniques et importantes, est l'incorporation importante soit de sodium, qui se révèle néfaste pour le système cardiaque, soit de potassium ou de lithium, qui sont toxiques et, de plus, peuvent entraîner des intoxications.

Les publications Chemical Abstracts Vol. 93, n° 37480 et Vol. 96, n° 155386 concernent respectivement l'administration simultanée de valproate de sodium ou de calcium et d'acide aminooxyacétique, ce dernier étant utilisé pour modifier l'action anticonvulsivante du valproate de sodium ou calcium. Il s'agit en fait de simples mélanges ou associations et non de produits de réaction, ces mélanges présentant les mêmes inconvénients que ceux des sels de métaux alcalins d'acide valproïque, de par la présence même de ceux-ci dans les mélanges. En fait, l'acide aminooxyacétique, du fait de la présence d'un seul groupement $NH_2$, est neutre ou très faiblement basique, et ne peut donc conduire à la formation d'un sel.

On notera également qu'il est connu suivant le brevet US-A-3.024.272 de synthétiser des sels d'acides organiques avec des acides aminés basiques, mais que ces sels ont une application tout à fait différente de celle des sels de l'invention, et ne laissent en aucun cas supposer qu'ils pourraient avoir une activité anticonvulsivante.

La présente invention a pour but de mettre au point un sel d'acide valproïque anticonvulsivant, solide et fortement soluble dans l'eau, ne présentant pas les inconvénients des sels de métaux alcalins. A cet effet, suivant l'invention, le sel est constitué par le produit de réaction de l'acide valproïque et au moins d'un acide aminé basique.

Avantageusement, le sel contient environ 1 à 5 moles, et de préférence environ 1 mole d'acide aminé basique par mole d'acide valproïque. Le ou les acides aminés basiques utilisés peuvent être naturels ou non comme, par exemple, l'arginine, la lysine, l'histidine, l'ornithine et la glycine. Les acides aminés basiques selon la présente invention peuvent renfermer un ou plusieurs centres asymétriques et peuvent donc exister sous les formes isomères optiquement actives. Il est bien entendu que l'invention couvre les deux formes épimères, telles que les formes lévogyre et dextrogyre, ainsi que leur mélange. Des exemples d'acides aminés basiques lévogyres et dextrogyres sont les D- et L- lysines et les D- et L-arginines.

L'invention concerne également la préparation de ces sels d'acide valproïque.

Selon une première façon de procéder, l'acide valproïque est mis en réaction avec une solution aqueuse contenant le ou les acides aminés basiques. Après réaction, l'excès d'acide est éliminé par lavage au moyen d'un solvant organique, l'eau étant ensuite séparée du mélange réactionnel ainsi obtenu par des méthodes de séparation appropriées, telles que par évaporation ou lyophilisation. Des exemples de solvants organiques sont les alcanes, les acétates d'alkyle, les solvants chlorés, et leurs mélanges.

Une autre façon de procéder consiste à mettre en réaction une solution ou dispersion aqueuse ou hydroorganique contenant le ou les acides aminés basiques avec une solution ou dispersion organique contenant l'acide valproïque, avec une dispersion aqueuse contenant l'acide valproïque ou bien avec l'acide valproïque tel quel, et à séparer le solvant du mélange réactionnel ainsi obtenu, par des méthodes de séparation appropriées, telles que par filtration, lyophilisation ou évaporation. Des exemples de solvants organiques utilisés pour dissoudre les acides aminés et l'acide valproïque sont les solvants organiques polaires, tels que les alcools, les glycols, les polyglycols, les cétones, le diméthylformamide et le diméthylsulfoxyde. On peut également utiliser des mélanges de tels solvants.

Une troisième façon de procéder consiste à mettre en contact un ou des sels de l'acide aminé ou des acides aminés basiques en solution aqueuse, organique ou hydroorganique avec de l'acide valproïque tel quel ou en solution organique, et à séparer le solvant du milieu réactionnel par des méthodes de séparation appropriées, telles que par évaporation, lyophilisation ou filtration. Un exemple de sel d'acide aminé est le carbonate de lysine. Des exemples de solvants de précipitation sont les acétates d'alkyle, l'éther sulfurique, le dioxane, le tétrahydrofuranne, les cétones, et leurs mélanges.

Suivant l'invention, dans les trois façons de procéder, le traitement susdit est effectué à une température de l'ordre de − 5 à 100 °C, et de préférence à une température voisine de 20 °C.

L'acide valproïque est un acide organique liquide peu soluble dans l'eau. Ainsi qu'on l'a déjà précisé, les sels se forment aisément avec les acides aminés basiques, tels que l'arginine, la lysine, l'histidine, l'ornithine et la glycine.

Les sels de l'acide valproïque parfaitement solubles dans l'eau requièrent de 0,5 à 5 molécules d'acide aminé par molécule d'acide valproïque.

En fait, les sels d'acide valproïque de l'invention répondent à la formule suivante :

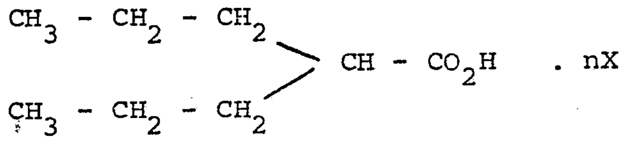

dans laquelle n, représentant le nombre de molécules d'acide aminé par rapport à 1 molécule d'acide valproïque, est compris entre 1 et 5, et de préférence compris entre 1 et 2, et X représente le ou les acides aminés.

Les sels de l'acide valproïque hydrosolubles de la présente invention peuvent donc être obtenus en utilisant des méthodes connues de préparation de sels, et, en particulier, par la mise en solution ou suspension aqueuse, hydroorganique ou organique d'un ou de plusieurs acides aminés, ou par la mise en solution ou suspension aqueuse, hydroorganique ou organique d'un ou de plusieurs sels d'acides aminés, à laquelle, tout en maintenant la température comprise entre 0 et 100 °C et de préférence de l'ordre de 20 °C, on ajoute, sous agitation, par petites portions la quantité d'acide valproïque requise, éventuellement sous forme de solution organique. On notera, à cet effet, que l'on peut inverser l'ordre d'addition des réactifs mis en présence.

Lorsque la solution est devenue limpide, on élimine le solvant du mélange réactionnel par des méthodes de séparation appropriées quelconques, telles que, par exemple, par filtration (solution hydroorganique ou organique), par lyophilisation (solution aqueuse) ou par chauffage modéré sous vide. On obtient ainsi une poudre soluble dans l'eau qui peut être utilisée pour la préparation de formes solides (comprimés, suppositoires, tablettes, granulés, dragées) et de formes injectables. Il va de soi que les solutions de sels d'acide valproïque préparés comme expliqué ci-dessus, peuvent être utilisées immédiatement sous forme injectable sans être lyophilisées au préalable, et pour autant que leur force ionique soit acceptable ou rendue telle.

Ci-dessous sont rassemblés quelques exemples, non limitatifs, de préparation des composés suivant l'invention.

## Exemple 1

A une solution aqueuse contenant 7,3 g de lysine base dans 50 ml d'eau, on ajoute progressivement et sous agitation 7,93 g d'acide valproïque. Après environ une heure de mélange, la solution aqueuse est lavée avec 20 ml de n-hexane. Après lyophilisation, on obtient environ 14,50 g de valproate de lysine instantanément soluble dans l'eau et présentant les caractéristiques suivantes : pH d'une solution à 10 % dans l'eau = 7,7 à 8,5 : point de fusion 152-154 °C, avec décomposition ; solubilité dans l'eau à 20 °C supérieure à 50 %. En procédant de la même façon avec l'arginine, l'histidine ou l'ornithine, on obtient les sels correspondants qui sont tous solubles dans l'eau.

## Exemple 2

A une solution de 14,6 g de lysine base dans 50 ml d'eau, on ajoute par petites portions 14,42 g d'acide valproïque dissous dans 100 ml de méthanol. Après environ une heure de réaction, la solution est évaporée à sec. La poudre blanche ainsi obtenue est remise en solution au moyen de 50 ml de méthanol, et après l'adjonction lente de 250 ml d'acétate d'éthyle sous agitation le sel cristallisé est filtré. Après séchage à 60 °C, on obtient environ 26 g de valproate de lysine (90 %) instantanément soluble dans l'eau.

## Exemple 3

A une suspension de 7,3 g de lysine base anhydre dans 25 ml de méthanol, on ajoute sous agitation 7,21 g d'acide valproïque. Une fois la solution devenue limpide, on la verse lentement dans 150 ml d'acétate d'éthyle sous agitation intense. Le précipité obtenu est filtré et lavé à l'acétate d'éthyle. On récupère environ 13,4 g (92 %) de valproate de lysine.

## Exemple 4

A une solution de 18,6 g de carbonate de lysine dans un mélange de 20 ml d'eau et 25 ml de méthanol, on ajoute par petites portions 15,84 g d'acide valproïque. Après environ une heure d'agitation, la solution limpide obtenue est filtrée et évaporée sous vide. Le résidu solide dissous dans 50 ml de méthanol est versé lentement dans 250 ml d'acétate d'éthyle sous agitation intense. Le sel de lysine cristallise sous forme de poudre blanche que l'on isole par filtration. Le valproate de lysine obtenu est instantanément soluble dans l'eau.

Les sels d'acide valproïque solubles dans l'eau de la présente invention exercent une activité anticonvulsivante remarquable. Les sels à base d'acide valproïque de l'invention peuvent être administrés en association avec divers excipients pharmaceutiques tels que des diluants, des gélifiants, des agents

conservateurs, des émulsionnants, des édulcorants et aromatisants, etc., et cela par voie orale, parentérale ou rectale.

Pour une administration orale, on utilisera des dragées, granulés, pastilles (pellets), tablettes, capsules, comprimés, solutions, sirops, émulsions contenant des additifs ou excipients classiques en pharmacie galénique. Ces formes galéniques peuvent libérer le principe actif de façon normale ou programmée dans le temps.

Pour l'administration parentérale, on utilisera tout véhicule approprié comme, par exemple, de l'eau stérile, une huile d'arachide ou de l'oléate d'éthyle.

Pour l'administration rectale, on utilisera des suppositoires, des capsules rectales, des solutions ou des gelées.

Le composé actif peut être administré seul ou en combinaison avec d'autres produits actifs ayant une activité similaire ou différente.

Les doses recommandées pour l'administration par la voie orale, rectale et intraveineuse sont, par exemple, de 100 mg à 6 g, avantageusement de 1 g à 3 g par jour.

On donne ci-après quelques exemples de formes solides contenant, comme principe actif, un des composés suivant l'invention.

Exemple 1

a) Formulation pour 1 comprimé :

| | |
|---|---|
| Acide valproïque (sel de lysine) : | 800 mg |
| Polyméthacrylate : | 25 mg |
| Stéarate de magnésium : | 1 mg |
| Acétate d'éthyle : | quantité suffisante |
| | 826 mg |

b) Procédé de fabrication :

1. Granuler ensemble, au moyen d'acétate d'éthyle, le sel de lysine de l'acide valproïque et le polyméthacrylate, tamiser et sécher.
2. Incorporer le stéarate de magnésium.
3. Comprimer à 0,826 g par comprimé.

Exemple 2

a) Formulation pour une dose de microgranules :

| | |
|---|---|
| Acide valproïque (sel de lysine) : | 800 mg |
| Hydroxypropylméthylcellulose : | 200 mg |
| Gomme laque : | quantité suffisante |
| Alcool éthylique : | quantité suffisante. |

b) Procédé de fabrication :

1. Granuler au moyen de la solution d'hydroxypropylméthylcellulose dans l'éthanol, le sel d'acide valproïque.
2. Traiter ce granulé avant dessication, par tout moyen adéquat pour en faire des microgranules plus ou moins sphériques.
3. Sécher les microgranules et les tamiser afin de sélectionner le diamètre souhaité.
4. Projeter, sur les microgranules ainsi obtenus, la gomme laque en solution alcoolique.
5. Sécher parfaitement les microgranules, les tamiser à nouveau et contrôler la cinétique de libération de l'acide valproïque, suivant le type désiré (retard, prolongé, etc.).
6. Mettre en gélules les microgranules obtenus.

On notera qu'une libération programmée du principe actif peut être obtenue par d'autres composants que la gomme laque, comme par exemple, l'éthylcellulose, l'acétophtalate de cellulose, les polyméthacrylates. Il est aussi évident que l'emploi de ces substances est indiqué lorsque l'on veut obtenir une libération retardée ou programmée de la substance active ; si une libération rapide doit être obtenue les étapes 4 et 5 peuvent être supprimées pour passer directement de l'étape 3 à l'étape 6.

On notera que les microgranules obtenus dans l'exemple 2 peuvent être présentés outre la mise en gélules, sous forme de comprimés, de suspension, de suppositoires ou toute autre forme pharmaceutique jugée acceptable.

De plus, les comprimés obtenus dans l'exemple 1 peuvent être réalisés de telle sorte que la libération du principe actif soit ou bien rapide ou bien retardée et/ou prolongée. Ceci s'obtiendra, notamment, en

modifiant les quantités du polymère méthacrylique et/ou en enrobant les comprimés ainsi qu'il est indiqué pour les microgranules de l'exemple 2.

Des études de toxicité aiguë par la voie orale sur le sel d'acide valproïque de l'exemple 1 ont donné les résultats suivants, exprimés en dose létale pour 50 % des animaux.

— Rats (2 sexes) : 2 980 mg par kg de poids corporel (soit 1 480 mg acide valproïque pur).

— Souris (2 sexes) : 2 211 mg par kg de poids corporel (soit 1 098 g en acide valproïque pur).

Les chiffres obtenus avec la substance suivant l'invention montrent une résorption rapide et importante. On ne remarque pas non plus d'effet létal retardé, ce qui constitue également un élément favorable dans ce type d'étude.

La substance administrée à 12 sujets sains donne des taux plasmatiques en acide valproïque identiques à ceux obtenus chez ces mêmes sujets après administration d'une dose équivalente de valproate de soude. Toutefois les écarts interindividuels semblent moins importants avec le sel de lysine qu'avec le sel de sodium.

**Revendications**

1. Sel d'acide valproïque hydrosoluble, d'activité anticonvulsivante, caractérisé en ce qu'il est constitué par le produit de réaction de l'acide valproïque et au moins d'un acide aminé basique.

2. Sel suivant la revendication, caractérisé en ce que l'acide aminé basique est un acide aminé lévogyre, dextrogyre ou un mélange de tels acides.

3. Sel suivant l'une ou l'autre des revendications 1 et 2, caractérisé en ce que l'acide aminé basique est choisi dans le groupe formé par l'arginine, la lysine, l'histidine, l'ornithine et la glycine.

4. Sel suivant l'une quelconque des revendications 1 à 3, caractérisé en ce qu'il contient environ 1 à 5 moles d'acide aminé basique par mole d'acide valproïque.

5. Sel suivant la revendication 4, caractérisé en ce qu'il contient environ 1 mole d'acide aminé basique par mole d'acide valproïque.

6. Procédé de préparation du sel d'acide valproïque hydrosoluble suivant l'une quelconque des revendications 1 à 5, caractérisé en ce que l'on traite l'acide aminé basique en solution aqueuse avec l'acide valproïque et en ce que l'on sépare l'eau du mélange réactionnel ainsi obtenu, par des méthodes de séparation appropriées quelconques, telles que par évaporation ou lyophilisation.

7. Procédé de préparation du sel d'acide valproïque hydrosoluble suivant l'une quelconque des revendications 1 à 5, caractérisé en ce que l'on traite l'acide aminé basique en solution ou suspension aqueuse, hydroorganique ou organique avec de l'acide valproïque en solution ou dispersion aqueuse, hydroorganique ou organique ou tel quel, et en ce que l'on sépare le solvant du mélange réactionnel ainsi obtenu par des méthodes de séparation appropriées quelconques, telles que par filtration, lyophilisation ou évaporation.

8. Procédé de préparation du sel d'acide valproïque hydrosoluble suivant l'une quelconque des revendications 1 à 5, caractérisé en ce que l'on traite un sel de l'acide aminé basique en solution ou suspension aqueuse, hydroorganique ou organique avec de l'acide valproïque tel quel ou en solution aqueuse, hydroorganique ou organique, et en ce que l'on sépare le solvant du mélange réactionnel ainsi obtenu par des méthodes de séparation appropriées quelconques, telles que filtration, lyophilisation ou évaporation.

9. Procédé suivant la revendication 8, caractérisé en ce que le sel d'acide aminé est le carbonate de lysine.

10. Procédé suivant l'une quelconque des revendications 6 à 9, caractérisé en ce que le traitement susdit est effectué à une température comprise entre – 5 °C et 100 °C.

11. Procédé suivant la revendication 10, caractérisé en ce que le traitement est effectué à une température de l'ordre de 20 °C.

12. Procédé suivant l'une quelconque des revendications 7 à 9, caractérisé en ce que le solvant organique utilisé pour dissoudre l'acide aminé ou son sel et l'acide valproïque est un solvant organique polaire ou un mélange de solvants organiques polaires.

13. Composition pharmaceutique comprenant, comme produit actif, au moins un sel d'acide valproïque hydrosoluble suivant l'une quelconque des revendications 1 à 5, associé à au moins un excipient approprié et/ou à au moins un autre agent thérapeutique.

**Claims**

1. A water-soluble valproic salt, characterized in that it is formed by the reaction product between valproic acid and at least one basic amino-acid.

2. A salt as claimed in claim 1, characterized in that the basic amino-acid is a levogyre amino-acid, a dextrogyre amino-acid or a mixture of said acids.

3. A salt as claimed in any of claims 1 and 2, characterized in that the basic amino-acid is selected from the group comprising arginine, lysine, histidine, ornithine and glycine.

4. A salt as claimed in any of claims 1 to 3, characterized in that it contains about 1 to 5 moles of basic amino-acid per mole of valproic acid.

5. A salt as claimed in claim 4, characterized in that it contains about 1 mole of basic amino-acid per mole of valproic acid.

6. A process for preparing the water-soluble valproic acid salt as claimed in any of claims 1 to 5, characterized in that the basic amino-acid in aqueous solution is treated with valproic acid and the water of the obtained reaction mixture is separated by any suitable separation method, such as evaporation or lyophilization.

7. A process for preparing the water-soluble valproic acid salt as claimed in any of claims 1 to 5, characterized in that the basic amino-acid in aqueous, hydro-organic or organic solution or suspension is treated with valproic acid as it is or in aqueous, hydro-organic or organic solution or suspension and the solvent is separated from the so obtained reaction mixture by any suitable separation method, such as by filtration, lyophilization or evaporation.

8. A process for preparing the water-soluble valproic acid salt as claimed in any of claims 1 to 5, characterized in that a salt of the amino-acid in aqueous, hydro-organic or organic solution or suspension is treated with valproic acid as it is or in aqueous, hydro-organic or organic solution or suspension and the solvent is separated from the so obtained reaction mixture by any suitable separation methods, such as by filtration, lyophilization or evaporation.

9. A process as claimed in claim 8, characterized in that lysine carbonate is the salt of amino-acid.

10. A process as claimed in any of claims 6 to 9, characterized in that said treatment is carried out at a temperature between − 5 °C and 100 °C.

11. A process as claimed in claim 10, characterized in that the treatment is carried out at a temperature of about 20 °C.

12. A process as claimed in any of claims 7 to 9, characterized in that the organic solvent used for dissolving or dispersing the amino-acid or its salt and the valproic acid is a polar organic solvent or a mixture of polar organic solvents.

13. A pharmaceutical composition, comprising as active product at least one water-soluble valproic acid salt according to any of claims 1 to 5, in association with at least a suitable excipient and/or at least another therapeutical agent.

**Ansprüche**

1. Wasserlösliches Salz der Valproinsäure mit krampflösender Wirkung, dadurch gekennzeichnet, daß es aus dem Reaktionsprodukt von Valproinsäure und wenigstens einer basischen Aminosäure besteht.

2. Salz nach Anspruch 1, dadurch gekennzeichnet, daß die basische Aminosäure eine linksdrehende Aminosäure, eine rechtsdrehende Aminosäure oder ein Gemisch aus solchen Aminosäuren ist.

3. Salz nach Anspruch 1 oder 2, dadurch gekennzeichnet, daß die basische Aminosäure aus der Gruppe ausgewählt ist, die Arginin, Lysin, Histidin, Ornithin und Glycin umfaßt.

4. Salz nach einem beliebigen der Ansprüche 1 bis 3, dadurch gekennzeichnet, daß es etwa 1 bis 5 Mol basische Aminosäure auf 1 Mol Valproinsäure enhält.

5. Salz nach Anspruch 4, dadurch gekennzeichnet, daß es etwa 1 Mol basische Aminosäure auf 1 Mol Valproinsäure enthält.

6. Verfahren zur Herstellung wasserlöslicher Salze der Valproinsäure nach einem beliebigen der Ansprüche 1 bis 5, dadurch gekennzeichnet, daß man die basische Aminosäure in wässriger Lösung mit Valproinsäure behandelt und von der so erhaltenen Reaktionsmischung das Wasser mittels beliebiger geeigneter Trennmethoden abtrennt, beispielsweise durch Verdampfen oder Gefriertrocknen.

7. Verfahren zur Herstellung wasserlöslicher Salze der Valproinsäure nach einem beliebigen der Ansprüche 1 bis 5, dadurch gekennzeichnet, daß man die basische Aminosäure in wässriger, hydroorganischer oder organischer Lösung oder Suspension mit in wässriger, hydroorganischer oder organischer Lösung oder Dispersion oder auch rein vorliegender Valproinsäure behandelt und von der so erhaltenen Reaktionsmischung das Lösungsmittel mittels beliebiger geeigneter Trennmethoden abtrennt, beispielsweise durch Filtrieren, Gefriertrocknen oder Verdampfen.

8. Verfahren zur Herstellung wasserlöslicher Salze der Valproinsäure nach einem beliebigen der Ansprüche 1 bis 5, dadurch gekennzeichnet, daß man ein Salz der basischen Aminosäure in wässriger, hydroorganischer oder organischer Lösung oder Suspension mit reiner oder in wässriger hydroorganischer oder organischer Lösung vorliegender Valproinsäure behandelt und von der so erhaltenen Reaktionsmischung das Lösungsmittel mittels beliebiger geeigneter Trennmethoden abtrennt, beispielsweise durch Filtrieren, Gefriertrocknen oder Verdampfen.

9. Verfahren nach Anspruch 8, dadurch gekennzeichnet, daß das Salz der Aminosäure Lysincarbonat ist.

10. Verfahren nach einem der Ansprüche 6 bis 9, dadurch gekennzeichnet, daß die Behandlung bei einer Temperatur zwischen − 5 °C und 100 °C erfolgt.

11. Verfahren nach Anspruch 10, dadurch gekennzeichnet, daß die Behandlung bei einer Temperatur im Bereich von etwa 20 °C erfolgt.

12. Verfahren nach einem der Ansprüche 7 bis 9, dadurch gekennzeichnet, daß das zum Lösen der Aminosäure oder deren Salz sowie der Valproinsäure verwendete organische Lösungsmittel ein polares organisches Lösungsmittel oder eine Mischung polarer organischer Lösungsmittel ist.

13. Pharmazeutische Zubereitung, die als Wirkstoff wenigstens ein wasserlösliches Salz der Valproinsäure nach einem beliebigen der Ansprüche 1 bis 5 enthält, in Verbindung mit wenigstens einem geeigneten Trägerstoff und/oder wenigstens einem weiteren therapeutischen Wirkstoff.